(19) Europäisches Patentamt
European Patent Office
Office européen des brevets

(11) **EP 4 738 267 A1**

(12) **EUROPEAN PATENT APPLICATION**

(43) Date of publication:
**06.05.2026 Bulletin 2026/19**

(51) International Patent Classification (IPC):
***G06T 12/10*** *(2026.01)*

(21) Application number: **25212371.6**

(52) Cooperative Patent Classification (CPC):
**G06T 12/10;** G06T 2211/412

(22) Date of filing: **30.10.2025**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **30.10.2024 CN 202411535055**

(71) Applicant: **Shanghai United Imaging Healthcare Co., Ltd.**
**Shanghai 201807 (CN)**

(72) Inventors:
• **TANG, Songsong**
**Shanghai, 201807 (CN)**
• **SHI, Lei**
**Shanghai, 201807 (CN)**
• **WANG, Beien**
**Shanghai, 201807 (CN)**
• **LIU, Zhongzhi**
**Shanghai, 201807 (CN)**
• **SUN, Youjun**
**Shanghai, 201807 (CN)**
• **DONG, Yun**
**Shangha, 201807 (CN)**

(74) Representative: **Murgitroyd & Company**
**165-169 Scotland Street**
**Glasgow G5 8PL (GB)**

(54) **IMAGE RECONSTRUCTION METHOD AND APPARATUS**

(57)     An image reconstruction method includes: acquiring raw scan data of a scan object by a scanning system, the scanning system including a detector, correcting the raw scan data based on at least one of scan-motion-associated correction factors to obtain corrected raw scan data, the scan-motion-associated correction factors being associated with scanning durations of a plurality of voxels of the scan object or scanning durations of a plurality of pixels of the detector, and performing image reconstruction on the corrected raw scan data to obtain a scan image of the scan object.

FIG. 16

# Description

## TECHNICAL FIELD

The present disclosure relates to the field of image reconstruction technologies, and in particular, to an image reconstruction method and apparatus.

## BACKGROUND

Single Photon Emission Computed Tomography (SPECT) is a relatively mature imaging technology in the field of nuclear medicine and has been widely used in clinical testing.

For scanning scenarios, in related technologies, uniform scanning of the detection object is mainly achieved by moving a probe and a bed to obtain a scan image of the detection object.

However, for non-uniform scanning requirements, the quality of the scan image obtained by methods in the related art is poor.

## SUMMARY

In a first aspect, an image reconstruction method is provided in the present disclosure, and the method includes:

acquiring raw scan data of a scan object by a scanning system, the scanning system including a detector;

correcting the raw scan data based on at least one of scan-motion-associated correction factors to obtain corrected raw scan data, the scan-motion-associated correction factors being associated with scanning durations of a plurality of voxels of the scan object or scanning durations of a plurality of pixels of the detector; and

performing image reconstruction based on the corrected raw scan data to obtain a scan image of the scan object.

In some embodiments, the raw scan data are acquired, on a condition that the scanning system, during a single scanning process, configures at least two different scanning durations respectively for the plurality of voxels of the scan object or the plurality pixels of the detector。

In some embodiments, the scanning system further includes a scanning bed, and acquiring the raw scan data of the scan object by the scanning system includes controlling the scanning bed to perform a movement along a horizontal axis to obtain the raw scan data.

In some embodiments, the movement includes at least one of a stepwise movement and a continuous movement.

In some embodiments, the scan-motion-associated correction factors include correction factors corresponding to different voxels of the scan object; and before correcting the raw scan data based on at least one of the scan-motion-associated correction factors, the method further includes:

acquiring start scan time points and end scan time points corresponding to the scanning durations of the plurality of voxels of the scan object; and determining, based on the start scan time points and the end scan time points, correction factors corresponding to the voxels of the scan object.

In some embodiments, determining, based on the start scan time points and the end scan time points, the correction factors corresponding to the voxels of the scan object includes:

calculating an average moving speed of the scanning bed based on the start scan time points and the end scan time points corresponding to the voxels; and

generating the correction factors corresponding to the voxels based on the start scan time points, the end scan time points, the average moving speed, and a nuclide half-life of a nuclide in the scan object.

In some embodiments, acquiring the raw scan data of the scan object by the scanning system includes controlling the detector to rotate relative to a horizontal axis to obtain the raw scan data.

In some embodiments, the scan-motion-associated correction factors include correction factors corresponding to different pixels of the detector. Correcting the raw scan data based on the at least one of scan-motion-associated correction factors to obtain the corrected raw scan data includes:

generating a likelihood function corresponding to the raw scan data based on the scanning durations and the correction factors; and correcting the raw scan data based on the likelihood function to obtain the corrected raw scan data.

In some embodiments, correcting the raw scan data based on the likelihood function to obtain the corrected raw scan data includes: on a condition that the likelihood function takes a maximum value, acquiring predicted scan data corresponding to the raw scan data, and using the predicted scan data as the corrected raw scan data.

In some embodiments, acquiring the raw scan data of the scan object by the scanning system includes:

acquiring a plurality of scan regions of the scan object;

determining scanning durations of the scanning system for the plurality of scan regions; and performing scanning based on the scanning durations of the scanning system for the plurality of scan regions to acquire the raw scan data.

In some embodiments, performing scanning based on the scanning durations of the scanning system for the plurality of scan regions to acquire the raw scan data includes:

> determining scanning moving speeds or scanning residence durations of the detector for the plurality of scan regions, based on the scanning durations of the scanning system for the plurality scan regions; and performing scanning based on the scanning moving speeds or the scanning residence durations to acquire the raw scan data.

In some embodiments, the scan regions are divided based on scanning requirements of the scan object.

In some embodiments, the scan regions are divided based on an anatomical structure of the scan object.

In a second aspect, an image reconstruction apparatus is further provided in the present disclosure, and the apparatus includes:

> a data acquisition module configured to acquire raw scan data of a scan object by a scanning system, the scanning system including a detector;
> a correction module configured to correct the raw scan data based on at least one of scan-motion-associated correction factors to obtain corrected raw scan data, the scan-motion-associated correction factors being associated with scanning durations of a plurality of voxels of the scan object or scanning durations of a plurality of pixels of the detector; and
> a reconstruction module configured to perform image reconstruction based on the corrected raw scan data to obtain a scan image of the scan object.

In a third aspect, a computer device is further provided in the present disclosure, which includes a memory and a processor. The memory stores a computer program, and the processor implements the steps of any one embodiment of the above-mentioned image reconstruction method when executing the computer program.

In a fourth aspect, a computer-readable storage medium is further provided in the present disclosure, having a computer program stored thereon. A processor implements the steps of any one embodiment of the above-mentioned image reconstruction method when executing the computer program.

In a fifth aspect, a computer program product is further provided in the present disclosure, including a computer program, which, when executed by a processor, implements the steps of any one embodiment of the above-mentioned image reconstruction method.

## BRIEF DESCRIPTION OF THE DRAWINGS

In order to more clearly illustrate the technical solutions in the embodiments of the present disclosure or related technologies, the following briefly introduces the drawings required for use in the embodiments of the present disclosure or related technical descriptions. Apparently, the drawings described below are only some embodiments of the present disclosure. For a person skilled in the art, other related drawings can be obtained based on these drawings without paying any creative efforts.

FIG. 1 is a diagram illustrating an application environment of an image reconstruction method in some embodiments.
FIG. 2 is a schematic flowchart illustrating an image reconstruction method in some embodiments.
FIG. 3 is a schematic diagram illustrating stepwise planar scanning in some embodiments.
FIG. 4 is a schematic diagram illustrating continuous variable-speed planar scanning in some embodiments.
FIG. 5 is a schematic flowchart illustrating an image reconstruction method in some embodiments.
FIG. 6 is a schematic diagram illustrating stepwise planar scanning in some embodiments.
FIG. 7 is a schematic diagram illustrating a curve of voxel counts versus scanning time points in some embodiments.
FIG. 8 is a schematic diagram illustrating continuous variable-speed planar scanning in some embodiments.
FIG. 9 is a schematic diagram illustrating a curve of voxel counts versus scanning time points in some embodiments.
FIG. 10 is a schematic flowchart illustrating an image reconstruction method in some embodiments.
FIG. 11 is a schematic diagram illustrating non-uniform tomographic scanning in some embodiments.
FIG. 12 is a schematic flowchart illustrating an image reconstruction method in some embodiments.
FIG. 13 is a schematic diagram illustrating non-uniform tomographic scanning in some embodiments.
FIG. 14 is a schematic diagram illustrating a curve of pixel counts versus scanning time points in some embodiments.
FIG. 15 is a schematic flowchart illustrating an image reconstruction method in some embodiments.
FIG. 16 is a schematic flowchart illustrating an image reconstruction method in some embodiments.
FIG. 17 is a block diagram illustrating a configuration of an image reconstruction apparatus according to one embodiment.
FIG. 18 is a diagram illustrating an internal configuration of a computer device in some embodiments.

## DETAILED DESCRIPTION OF THE EMBODIMENTS

In order to make the purpose, technical solutions and advantages of the present disclosure more clear, the disclosure is further described in detail below with reference to the accompanying drawings and examples. It should be understood that the specific embodiments described herein are only used to explain the present disclosure and are not intended to limit the present disclosure. Apparently, the embodiments described are only part of the embodiments of the present disclosure, not all of them. Based on the embodiments in the present disclosure, all other embodiments obtained by a person skilled in the art without making creative efforts are within the scope of protection of the present disclosure.

Before introducing specific contents of the present disclosure in detail, a brief introduction to the background technology of the present disclosure is first given.

In clinical scanning scenarios, for common dual-probe SPECT, the movement of the detector and scanning bed can be either stepwise movement or continuous movement. Stepwise movement refers to keeping a probe of the detector stationary at each predetermined acquisition position and collecting data for a same duration, while moving step by step to a next predetermined acquisition position for collection between a plurality of predetermined acquisition positions. Continuous movement refers to the probe maintaining a constant speed and moving continuously during a scanning process while collecting scan data for the scan regions.

Whether it is stepwise movement or continuous movement, taking planar scanning as an example, the essence is to scan different voxels of the scan object with the same scanning duration. For example, for a scan object with a height of 180 cm, on a condition that the scanning speed is 15 cm/min, a required scanning time is 12 minutes, which makes the scan object's comfort and scanning efficiency low. However, during a scanning process, it is often desirable to increase an acquisition time or counts for the lesion region so that the scanning process of the lesion region is more detailed, and other areas except the lesion region can be scanned quickly. On a condition that a non-uniform acquisition is used for clinical scanning, the quality of the scan image is poor.

To address the above issues, an image reconstruction method and apparatus are provided in the present disclosure, which corrects non-uniform scan data based on specific scanning requirements and reconstructs the image based on the corrected non-uniform scan data, thereby improving the quality of the scan image. Of course, the technical solutions provided in the embodiments of the present disclosure are not limited to solving only the above issues and also have other technical effects, which can be specifically described in the following embodiments. The technical solutions of the present disclosure are now described in detail.

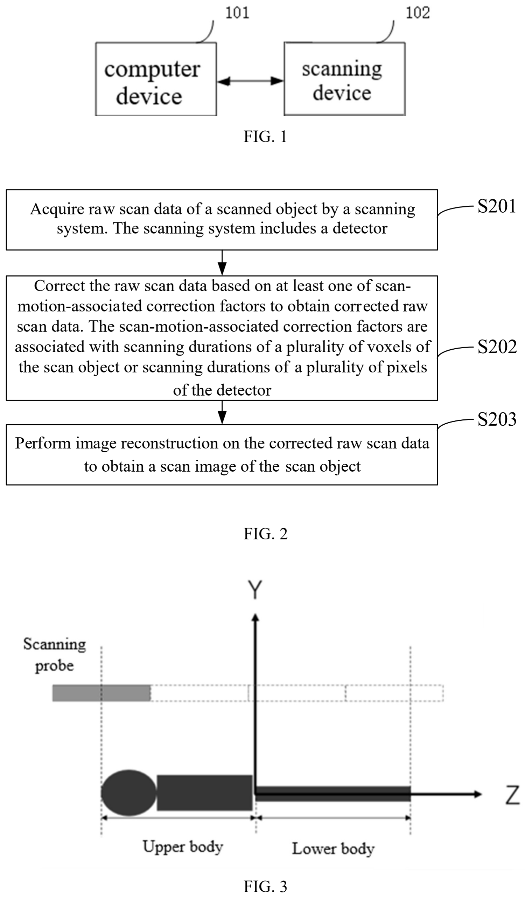

The image reconstruction method provided in the embodiment of the present disclosure can be applied in an application environment shown in FIG. 1. The application environment includes a computer device 101 and a scanning device 102, and the computer device 101 and the scanning device 102 communicate with each other. The scanning device 102 performs non-uniform scanning on the scan object at different moving speeds to obtain raw scan data, and sends the raw scan data to the computer device 101. For example, the scanning device 102 may include a detector. The computer device 101 can correct the raw scan data based on scan-motion-associated correction factors, such as the correction factors corresponding to different voxels of the scan object, to obtain corrected raw scan data, and perform image reconstruction based on the corrected raw scan data to obtain a scan image of the scan object.

In an exemplary embodiment, as shown in FIG. 2, an image reconstruction method is provided, which is described by taking the method applied to the computer device in FIG. 1 as an example. The method includes the following steps S201 to S203.

In step S201, raw scan data of a scan object are acquired by a scanning system. The scanning system includes a detector.

The detector can scan the scan object at a constant acceleration or a variable acceleration relative to the scan object. For example, the constant acceleration means that a speed gradually increases or decreases, and the variable acceleration means that the acceleration gradually increases or decreases. Alternatively, the scan object can be divided into different scan regions, the detector scans each of the different scan regions at a variable speed. For example, the corresponding scan regions can be scanned at different speeds. The scanning speed within each scan region can be the same, and the scanning speeds in different scan regions can be different. For example, the scan region can be the head, abdomen, and lower limbs of the scan object, each belonging to a different scan region. The lesion region and non-lesion region of the scan object can also belong to different scan regions. The detector can also scan different preset acquisition positions of the scan object with different scanning residence durations. The raw scan data can be thus obtained by non-uniform scanning according to some embodiments.

In the embodiment of the present disclosure, after the mobile scanning device performs non-uniform scanning on the scan object at a variable speed, the scan data are stored in a scan database. At this time, the computer device can obtain the raw scan data of the scan object from the scan database based on identification information of the scan object.

Optionally, the computer device may determine a plurality of scanning speeds for the scan object based on the physician's scanning requirements for the scan object. Based on the scanning requirements and the plurality of scanning speeds for the scan object, a control instruction may be generated. The control instruction is configured to control the mobile scanning device to perform a non-uniform scanning of the scan object, thereby acquiring the raw scan data. The embodiments of the present disclosure do not limit the method for acquiring the raw scan data of the scan object.

In step S202, the raw scan data are corrected based on at least one of scan-motion-associated correction factors to obtain corrected raw scan data. The scan-motion-associated correction factors are associated with scanning durations of a plurality of voxels of the scan object or scanning durations of a plurality of pixels of the detector.

In the embodiments of the present disclosure, before scanning a scan object using a mobile device, a nuclide is injected into the scan object. Taking planar scanning as an example, if during a scanning process, due to non-uniform scanning, the effective acquisition duration of the detector for different voxels is inconsistent, the nuclide decay degree of each voxel will be different, which is manifested as a count deviation of the raw scan data, i.e., the accuracy of the raw scan data is poor. Taking tomographic scanning as an example, due to the non-uniformity of the scanning movement, such as the change of the rotation speed of detector, the effective acquisition duration of different pixels of the detector for the scan object is inconsistent, which also leads to poor accuracy of the raw scan data. Therefore, counts of the raw scan data need to be corrected to ensure its accuracy. For example, when the scanning system is SPECT, the nuclide injected into the scan object is the radioactive element technetium-99m (Tc-99m). An injected amount of this nuclide is relatively small and is excreted through normal metabolism.

Specifically, non-uniform scanning can be divided into two situations. One is that the scan object moves along with a scanning bed of the scanning system for non-uniform scanning. The other is that the detector of the scanning system moves for non-uniform scanning. That is, a mobile scanning device can be a scanning bed or a detector. Taking the continuous variable-speed movement of the mobile scanning device as an example, a scan region corresponding to the case where the moving speed of the mobile scanning device is fast has a short acquisition duration, the influence of the nuclide decay time is small, and a count is relatively large. A scan region corresponding to the case where the moving speed of the mobile scanning device is slow has a long acquisition duration, the influence of the nuclide decay time is large, and a count is relatively small. Therefore, the number of events of different pixels in a scan image varies at different moving speeds. To ensure that the quality of different pixels in the corrected scan image is at the same count level, the scan-motion-associated correction factors are also different under different scanning durations of the plurality of voxels of the scan object or different scanning durations of the plurality of pixels of the detector. Based on this, the computer device can use the corresponding scan-motion-associated correction factors to correct the raw scan data, thereby obtaining the corrected raw scan data.

In step S203, image reconstruction is performed based on the corrected raw scan data to obtain a scan image of the scan object.

In an embodiment of the present disclosure, after obtaining the corrected raw scan data, the computer device can input the corrected raw scan data into a predetermined image reconstruction model, and use the image reconstruction model to analyze the raw scan data to reconstruct a scan image of the scan object. The image reconstruction model can be a fully convolutional network (e.g., a V-Net network, a U-Net network, etc.) or a generative adversarial network (e.g., a pix2pix network, a WGAN network, etc.).

Optionally, the computer device may also reconstruct the corrected raw scan data using an image reconstruction algorithm to obtain a scan image of the scan object. For example, the image reconstruction algorithm may be a back projection method, a Fourier transform reconstruction method, a filtered back projection reconstruction method, a convolution back projection method, or an iterative reconstruction algorithm.

In the above-mentioned image reconstruction method, the raw scan data of the scan object are obtained by the scanning system. The scanning system includes a detector. The raw scan data are corrected based on at least one of scan-motion-associated correction factors to obtain corrected raw scan data. The scan-motion-associated correction factors are associated with scanning durations of the plurality of voxels of the scan object or scanning durations of the plurality of pixels of the detector. Image reconstruction is performed based on the corrected raw scan data to obtain a scan image of the scan object. Since the raw scan data obtained by non-uniform scanning is inaccurate, the scan image obtained by direct reconstruction is also inaccurate. Based on this, the method of the embodiments obtains scan-motion-associated correction factors related to the scanning duration of the plurality of voxels of the scan object or the scanning duration of the plurality of pixels of the detector, which can accurately correct the raw scan data obtained by non-uniform scanning in a targeted manner, and make the corrected raw scan data more accurate through correction, so as to improve the quality of the reconstructed scan image. At the same time, it can also reduce the waiting time during the scanning process and improve scanning efficiency.

In some embodiments, the raw scan data are acquired, on a condition that the scanning system, during a single scanning process, configures at least two different scanning durations respectively for the plurality of voxels of the scan object or the plurality pixels of the detector. That is, the raw scan data are obtained by the scanning system performing non-uniform scanning on the scan object.

Assuming that the scanning system includes a scanning bed and a detector, and the scanning bed or the detector acts as the scanning mobile device, the specific content of acquiring the raw scan data of the scan object by the scanning system is introduced in some embodiments. Acquiring the raw scan data of the scan object by the scanning system includes controlling the scanning bed to perform a movement along a horizontal axis to obtain the raw scan data.

In the embodiments of the present disclosure, to enable variable-speed scanning of the mobile scanning device, it is necessary to ensure that the detector and the scanning bed in the mobile scanning device move at relative different speeds. In this case, the detector can be controlled to move along a length direction of the scanning bed or rotate relative to the length direction of the scanning bed, or the scanning bed can be controlled to move, so that different scan regions of the scan object enter a scanning field of view of the detector in sequence, for example, moving along a horizontal axis, to obtain the raw scan data. It should be noted that to ensure the quality of the raw scan data, the detector and the scanning bed cannot be controlled to move simultaneously.

In the above-mentioned image reconstruction method, the detector is controlled to scan the scan object at a variable speed relative to the scanning bed to obtain the raw scan data. This method controls the relative movement of the detector and the scanning bed to achieve a variable-speed scanning of the scan object, thereby ensuring that the raw scan data are obtained after non-uniform scanning by the mobile scanning device.

The above embodiments mentioned that the non-uniform scanning methods include non-uniform planar scanning and non-uniform tomographic scanning. The following describes these two scanning methods one by one through different embodiments.

First, the non-uniform planar scanning method is introduced. In some embodiments, the scanning system further includes a scanning bed. Acquiring the raw scan data of the scan object by the scanning system includes: controlling the scanning bed to perform a movement along a horizontal axis to obtain the raw scan data.

Specifically, on a condition that the detector is fixed in position, controlling the scanning bed to perform a non-uniform planar movement along the horizontal axis, and the raw scan data are acquired when the scanning bed enters a scanning range of the detector.

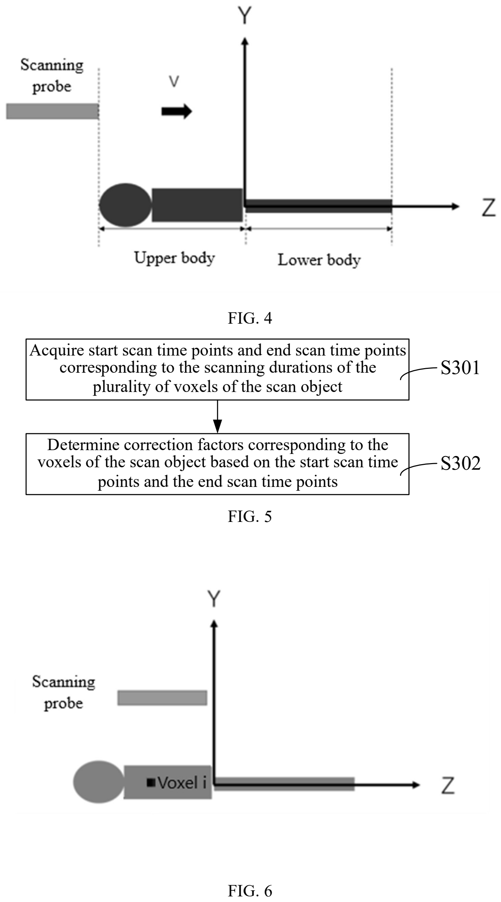

For the planar scanning process, the position of the detector in the mobile scanning device needs to be fixed, and the scanning bed is moved to scan the scan object on the scanning bed to obtain the raw scan data of the scan object. It should be noted that non-uniform planar movement can include at least one of a non-uniform stepwise movement and a continuous variable-speed movement. The non-uniform planar scanning method can be further divided into non-uniform stepwise planar scanning and continuous variable-speed planar scanning. FIG. 3 shows a schematic diagram of stepwise planar scanning. The detector is set at a fixed position, and the scanning bed moves below the detector. The upper and lower parts of the scan object each correspond to a scan region. For example, each scan region corresponds to a scanning duration. Scanning durations for the upper and lower parts of the body are completely different. Alternatively, the scan regions corresponding to the upper and lower parts of the body can be scanned according to different scanning durations respectively. FIG. 4 shows a schematic diagram of continuous variable-speed planar scanning. The detector is set at a fixed position, and the scanning bed moves below the detector. The upper body and lower body of the scan object correspond to one scan region respectively. Taking each scan region corresponding to one scanning speed as an example, a scanning speed for the upper body can be completely different from that for the lower body. Alternatively, for the scan regions corresponding to the upper body or the lower body, they can be further scanned at a non-uniform speed respectively.

In the embodiments of the present disclosure, the control instructions sent by the computer device to the mobile scanning device include control instructions for the detector and the scanning bed. Therefore, in the case of non-uniform scanning, the detector remains fixed after receiving the control instruction. In this case, the computer device sends control instruction to the scanning bed, controlling the scanning bed's movement within the scanning range of the detector. This movement is non-uniform and occurs in a planar manner. The detector acquires scan data from the scan object during the movement of the scanning bed. Once the movement of the scanning bed is complete, the acquisition process of the detector ends, and the detector sends the acquired raw scan data to the computer device.

In the above-mentioned image reconstruction method, with the detector position fixed, the scanning bed is controlled to perform a non-uniform planar movement relative to the detector to obtain raw scan data. During the non-uniform planar scanning process, this method fixes the detector position and limits the scanning bed's moving speed, allowing the detector to scan different scan regions of the scan object in a non-uniform acquisition manner, thereby accurately acquiring raw scan data of the scan object.

Assuming that the scan-motion-associated correction factors include correction factors corresponding to different voxels of the scan object, in some embodiments, as shown in FIG. 5, before correcting the raw scan data based on at least one of the scan-motion-associated correction factors, obtaining the correction factors corresponding to different voxels of the scan object includes the following steps S301 to S302.

In step S301, start scan time points and end scan time points corresponding to the scanning durations of the plurality of voxels of the scan object are acquired.

During a non-uniform planar scanning process, the start scan time points refer to the time points when the voxels of the scan object move to the start position of the detector, and the end scan time points refer to the time points when the voxels of the scan object move to the end position of the detector.

In an embodiment of the present disclosure, while a mobile scanning device is scanning the scan object, it can record the scanned position and corresponding scan time points, and store this recorded information and the raw scan data in a scan database. Therefore, a computer device can search the scan database based on the identification information of the scan object to obtain the start and end scan time points corresponding to the voxels of the scan object during the non-uniform planar scanning process.

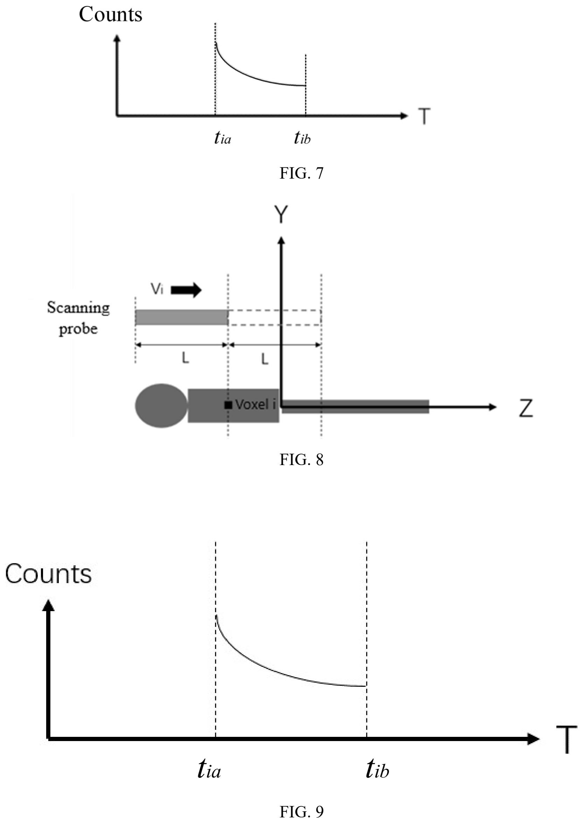

FIG. 6 shows a schematic diagram of stepwise scanning. As can be seen from the FIG. 6, any unit volume on the scan object can be considered as a voxel $i$. FIG. 7 shows a schematic diagram of a curve of counts versus scanning time points, where the ordinates represent the detector counts at the voxels, and the abscissas represent time changes during the detector scanning process. The start scan time point of the voxel is $t_{ia}$, and the end scan time point is $t_{ib}$. A scanning duration $D_i$ for voxel $i$ can be expressed as formula (1):

$$D_i = t_{ib} - t_{ia} \qquad (1)$$

FIG. 8 is a schematic diagram of continuous variable-speed planar scanning, showing the position of voxel $i$ and the length $L$ of the detector. FIG. 9 is a schematic diagram of the curve of counts versus scanning time points. The abscissas and ordinates in FIG. 9 are the same as those in FIG. 7. For any voxel $i$ on the scan object, the start scan time point corresponding to voxel $i$ just entering the scanning range of the detector is $t_{ia}$, and the start scan time point corresponding to voxel $i$ just leaving the scanning range of the detector is $t_{ib}$.

In step S302, correction factors corresponding to the voxels of the scan object are determined based on the start scan time points and the end scan time points.

In an embodiment of the present disclosure, after obtaining the start scan time point and the end scan time point corresponding to each voxel of the scan object, the computer device can calculate the scanning speed of each voxel based on the scanning duration between the start scan time point and the end scan time point of the voxel. Furthermore, based on the theoretical relationship between scanning speed and the correction factor, the correction factor corresponding to the voxel is determined. In this manner, the correction factor corresponding to each voxel of the scan object can be obtained.

It should be noted that for long-half-life nuclides (scanning duration is very short relative to the half-life), on a condition that a scan region is uniform, all voxels within the scan region will have the same scanning speed and the corresponding correction factors will also be the same. On a condition that a scan region is non-uniform, different voxels within the scan region will have different scanning speeds and the corresponding correction factors will also be different.

In the above-mentioned image reconstruction method, the start scan time points and the end scan time points corresponding to the voxels of the scan object during a non-uniform planar scanning process are obtained. The correction factors corresponding to the voxels of the scan object are determined based on the start scan time points and the end scan time points. By analyzing the start scan time points and the end scan time points corresponding to the voxels of the scan object, this method can accurately determine the scanning speeds of the voxels based on the analysis results, and thus accurately determine the correction factors corresponding to the voxels based on the scanning speeds.

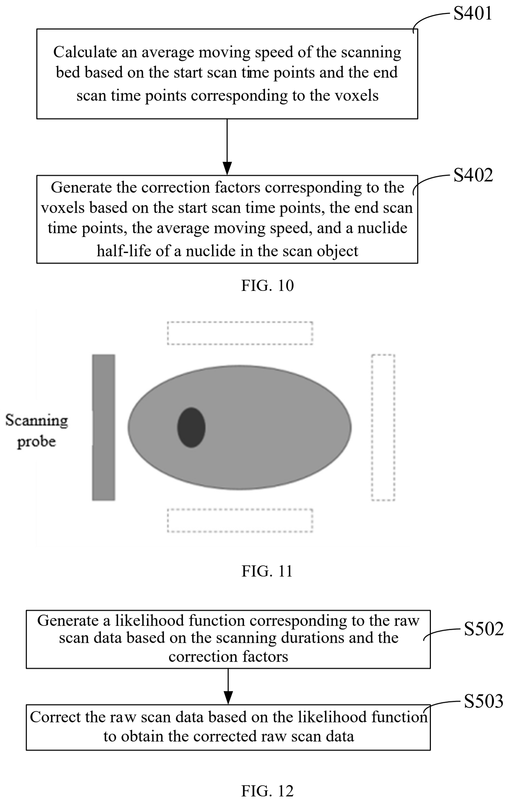

In some embodiments, as shown in FIG. 10, determining the correction factors corresponding to the voxels of the scan object based on the start scan time points and the end scan time points includes steps S401 to S402.

In step S401, an average moving speed of the scanning bed is calculated based on the start scan time points and the end scan time points corresponding to the voxels.

In the embodiment of the present disclosure, taking a planar scanning of the scanning system performing the continuous variable-speed movement of the mobile scanning device as an example, for any voxel, the distance it moves during the non-uniform planar scanning process is the detector length $L_{det}$, and the scanning duration is a time difference between the scan time point and the end scan time point. Therefore, the computer device can acquire a relationship between the scanning duration, the scan time point and the end scan time point, and use a ratio of the detector length to the scanning duration as the average moving speed of the scanning bed. The average moving speed $v_i$ can be expressed as formula (2):

$$v_i = L_{det} / (t_{ib} - t_{ia}) \qquad (2)$$

In step S402, the correction factors corresponding to the voxels are generated based on the start scan time points, the end scan time points, the average moving speed, and a nuclide half-life of a nuclide in the scan object.

In the embodiment of the present disclosure, each voxel corresponds to a correction factor. On a condition that the average moving speeds $v_i$ and the start scan time points $t_{ia}$ corresponding to the voxels in a scan region are the same respectively, the voxels in the scan region correspond to one correction factor. After obtaining the start scan time point, the end scan time point, and the average moving speed of the voxel, the correction factor corresponding to the voxel can be determined based on a calculation formula of the correction factor.

The calculation formula for the correction factor $f_i$ corresponding to any voxel can be expressed as formula (3):

$$f_i = \frac{(\frac{L}{v_i}\ln\;)\exp(\frac{t_{ia}-T_{cal}}{T_{1/2}}\ln2)}{T_{1/2}[1-\exp(-\frac{L_{det}}{v_i*T_{1/2}}\ln\;)]} \qquad (3)$$

where $T_{cal}$ refers to the initial scanning time of the detector, and $T_{1/2}$ refers to the half-life of the nuclide. The initial scanning time point of the detector refers to the time point when the detector first performs the nuclide scanning operation during the entire continuous movement process, which is used to provide an initial time reference for accurately calculating the nuclide decay process later.

In the above-mentioned image reconstruction method, the average speed of the scanning bed is calculated based on start scan time points and end scan time points corresponding to the voxels. Correction factors corresponding to the voxels are generated based on the start scan time points and end scan time points, the average speed, and the half-life of the nuclide in the scan object. This method accurately determines a time it takes for any voxel to pass through the detector based on the start scan time point and end scan time point corresponding to the voxel. This allows the average speed of the scanning bed to be accurately determined based on the length and the time of the detector, and thus accurately generates the correction factor corresponding to the voxel. In specific implementation, the correction factors can be introduced into a forward projection model, and when constructing a model corresponding to events emitted by any voxel being received by any detector pixel, the correction factors are considered at the same time for calculation, so that correction can be realized.

Next, the specific content of non-uniform tomographic scanning is introduced. In some embodiments, non-uniform scanning includes non-uniform tomographic scanning. Acquiring the raw scan data of the scan object by the scanning system includes: controlling the detector to rotate relative to a horizontal axis to obtain the raw scan data.

Specifically, on a condition that the scanning bed is fixed in position, controlling the detector to perform a non-uniform movement at different angles of the scanning bed to obtain the raw scan data.

For tomographic scanning, the scanning bed in the mobile device must be fixed in position. The detector rotates to scan the scan object on the scanning bed to generate raw scan data. FIG. 11 shows a schematic diagram of non-uniform tomographic scanning. As can be seen from FIG. 11, the detector rotates around the scan object at a continuously variable speed, i.e., an angular speed of the detector varies with changes of an angle relative to the scan object, presenting non-uniform rotation for acquisition. The darker areas in the FIG. 11 represent a region of interest.

In the embodiments of the present disclosure, control instructions sent by the computer device to the mobile scanning device include control instructions for the detector and the scanning bed. Therefore, in the case of a non-uniform scan is a non-uniform tomographic scanning, the scanning bed remains fixed in position after receiving the control instruction. In this case, the computer device sends control instruction to the detector, causing it to rotate around the scanning bed, and this rotation is non-uniform. During this rotation, the detector acquires scan data of the scan object on the scanning bed. Once the detector completes its movement, the acquisition process ends, and the detector sends the acquired raw scan data to the computer device.

In the above-mentioned image reconstruction method, while the scanning bed is fixed in position, the detector is controlled to perform a non-uniform movement at different angles along the scanning bed to obtain raw scan data. During the non-uniform tomographic scanning process, this method fixes the position of the scanning bed and, by limiting the moving speed of the detector, enables the detector to scan the scan object at different scanning angles using a non-uniform acquisition method, thereby accurately acquiring the raw scan data of the scan object.

Assuming that the scan-motion-associated correction factors include correction factors corresponding to different pixels of the detector, in some embodiments, as shown in FIG. 12, a specific content of correcting the raw scan data based on the at least one of scan-motion-associated correction factors to obtain the corrected raw scan data is described, and the specific content includes the following steps S502 to S503.

In the embodiments of the present disclosure, taking a pixel of the detector as an example, its scanning duration can include a scanning duration for one voxel of the scan object, i.e., a duration during which the scanning range of the detector covers the voxel. For any pixel of the detector, the computer device can acquire the start scan time point and the end scan time point corresponding to the scanning duration of the pixel. Assuming that the start scan time point of the pixel is $t_{ia}$ and the end scan time point of the pixel is $t_{ib}$, then a relationship between the scanning duration $D_i$ of pixel $i$, the start scan time point $t_{ia}$, and the end scan time point $t_{ib}$ can be expressed as formula (4):

$$D_i = t_{ib} - t_{ia} \qquad (4)$$

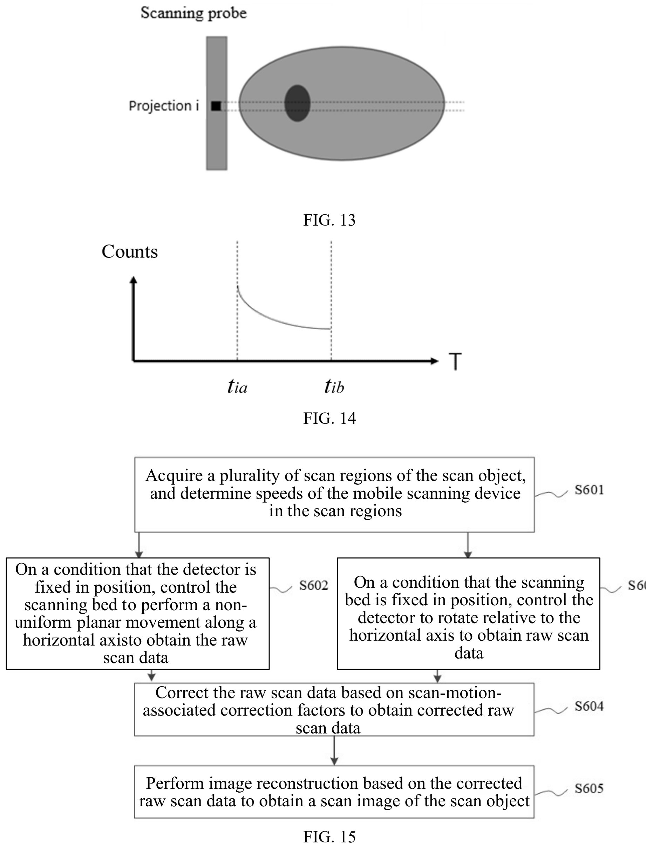

FIG. 13 is a schematic diagram of non-uniform tomographic scanning, showing a projection of the region of interest on the detector. FIG. 14 is a schematic diagram of a curve of pixel counts versus scanning time points. Similarly, horizontal and vertical coordinates in FIG. 14 are the same as those in FIG. 7. For any pixel $i$ on the scan object, the start scan time point of pixel $i$ on the detector is $t_{ia}$, and the end scan time point of pixel $i$ is $t_{ib}$.

In step S502, a likelihood function corresponding to the raw scan data is generated based on the scanning durations and the correction factors.

In the embodiment of the present disclosure, for any pixel, the correction factor $a_i$ corresponding to the pixel can be expressed as formula (5):

$$a_i = a_0 \frac{T_{1/2}[1-e^{(-\frac{D_i}{T_{1/2}}\ln 2)}]}{(\frac{D_i}{v_i}\ln \, )\exp(\frac{t_{ia}-T_{cal}}{T_{1/2}}\ln 2)} \qquad (5)$$

where $T_{cal}$ refers to an initial scanning time of any pixel of the detector, $T_{1/2}$ refers to a half-life of the nuclide, $a_0$ refers to an initial activity, for uniform tomographic scanning, $D_i$ can be a constant, and for non-uniform tomographic scanning, $D_i$ can take any value. For example, the scanning duration $D_i$ can be predetermined, and the scanning system controls the start scan time point $t_{ia}$ and the end scan time point $t_{ib}$ based on the scanning duration $D_i$. For continuous tomographic scanning, the start scan time point $t_{ia}$ refers to an initial acquisition time of a pixel at a certain angle, and the end scan time point $t_{ib}$ refers to a last acquisition time of the pixel at the angle. For stepwise tomographic scanning, the start scan time point $t_{ia}$ refers to an initial acquisition time of a pixel at a predetermined acquisition position, and the end scan time point $t_{ib}$ refers to a last acquisition time of the pixel at the predetermined acquisition position.

A likelihood function $L$ corresponding to the raw scan data can be expressed as formula (6):

$$L(x) = \sum_{i=1}^{M} [y_i \log(\bar{y}_i) - \bar{y}_i] \qquad (6)$$

where $x$ refers to a scan image, $y$ refers to a projection, $y_i$ refers to a true value of an $i$-th projection, and $\bar{y}_i$ refers to an expected value of the $i$-th projection, which can be expressed as formula (7):

$$y_i = A_i \sum_{j=1}^{N} p_{ij}x_j + s_i \qquad (7)$$

where $x_j$ refers to a $j$-th voxel, and $p_{ij}$ refers to a system matrix, i.e., a probability that the gamma ray emitted by the $j$-th voxel is received by the $i$-th projection. The predicted scan data can include the above expected value.

The total number of pixels in the scan image is $N$, the total number of projection data is $M$, and the product of the scanning time of the $i$-th projection and the correction factor $A_i$ can be expressed as formula (8):

$$A_i = a_i D_i \qquad (8)$$

In step S503, the raw scan data are corrected based on the likelihood function to obtain the corrected raw scan data.

In an embodiment of the present disclosure, after obtaining the likelihood function, the computer device can determine the predicted scan data corresponding to the raw scan data under predetermined conditions of the likelihood function based on predetermined constraints, and use the predicted scan data as the corrected raw scan data. Specifically, the likelihood function processes the raw scan data to obtain the corrected raw scan data, and then performs image reconstruction based on the corrected raw scan data to generate the scan image $x$. On a condition that the likelihood function takes the maximum value, it indicates that the corrected raw scan data is an optimal correction result of the raw scan data.

In the above-mentioned image reconstruction method, a likelihood function corresponding to the raw scan data is generated based on the scanning duration and a correction factor. The raw scan data are corrected based on the likelihood function to obtain corrected raw scan data. This method calculates the scanning duration for each pixel of the detector and, based on scanning duration and correction factors, accurately obtains the likelihood function corresponding to the raw scan data. This method then accurately performs correction based on the likelihood function, resulting in higher accuracy for the corrected raw scan data.

Next, a specific correction process is introduced through an embodiment. The specific content of correcting the raw scan data based on the likelihood function to obtain the corrected raw scan data includes:
On a condition that the likelihood function takes a maximum value, acquiring predicted scan data corresponding to the raw scan data, and the predicted scan data are used as the corrected raw scan data.

In the embodiment of the present disclosure, assuming that the likelihood function $L$ is the largest, an estimated value of the scan data, i.e., the predicted scan data, can be obtained. The predicted scan data can be expressed as formula (9):

$$(\hat{x}) = \arg\max(L) \qquad (9)$$

In the above-described image reconstruction method, on a condition that the likelihood function takes its maximum value, predicted scan data corresponding to the raw scan data are obtained, and the predicted scan data are used as the corrected raw scan data. This method can accurately analyze the raw scan data on a condition that the likelihood function takes its maximum value to accurately obtain the predicted scan data corresponding to the raw scan data, thereby accurately obtaining the corrected raw scan data.

Next, a process of acquiring the raw scan data of the scan object by the scanning system is described through an embodiment. The process includes the following steps:

acquiring a plurality of scan regions of the scan object;

determining scanning durations of the scanning system for the plurality of scan regions; and

performing scanning based on the scanning durations of the scanning system for the plurality of scan regions to acquire the raw scan data.

The scan regions can be divided based on scanning requirements of the scan object. The scanning requirements of the scan object refer to clinical scanning requirements determined by the doctor after preliminary analysis of the scan object. For example, the clinical scanning requirements may be: since an anatomical structure of the lower limb of the scan object is simple, only the general lesion needs to be seen, which means that the lower limb needs to be scanned faster.

In an embodiment of the present disclosure, after obtaining a scanning requirement for a scan object, the computer device can divide a scan region based on the number of different scanning durations in the scanning requirement, thereby obtaining a plurality of scan regions. The computer device can then obtain the scanning speed corresponding to each scan region based on a scanning duration and use the scanning speed as the moving speed of the scan region. For example, on a condition that the scanning requirement specifies a scanning speed of V1 for a upper body and a scanning speed of V2 for a lower body, the scan object can be divided into two scan regions, one corresponding to the upper body and the other to the lower body.

Alternatively, the scan object may be scanned at a constant acceleration or variable acceleration, and the computer device may determine the scanning speeds at different positions of the scan object based on the scanning rule. In the case of constant acceleration or variable acceleration, the scanning speeds for different scan regions are completely different.

In the above-mentioned image reconstruction method, a plurality of scan regions of the scan object are acquired. scanning durations of the scanning system for the plurality of the scan regions are determined. And scanning is performed based on the scanning durations of the scanning system for the plurality of scan regions to acquire raw scan data. This method divides different scan regions of the scan object targeted by the mobile scanning device, acquires the scanning durations of the mobile scanning device in each scan region, and can adjust the scanning durations for different scan regions to meet scanning requirements.

In some embodiments, performing scanning based on the scanning durations of the scanning system for the plurality of scan regions to acquire the raw scan data includes: determining scanning moving speeds or scanning residence durations of the detector for the plurality of scan regions, based on the scanning durations of the scanning system for the plurality scan regions, and performing scanning based on the scanning moving speeds or the scanning residence durations to acquire the raw scan data. Specifically, for a scan region with a longer scanning duration, in the case of continuous scanning, the scanning moving speed of the detector is slower, and in the case of stepwise scanning, the scanning residence duration of the detector is longer.

In some embodiments, the scan regions are divided based on the scanning requirements of the scan object. The scan regions are divided based on the scanning requirements, and the speed of the mobile scanning device in each scan region can be determined specifically based on the scanning requirements.

In some embodiments, the scan regions are divided based on an anatomical structure of the scan object. Based on the anatomical structure of the scan object, the scan regions are obtained, and the speed of the mobile scanning device in each scan region can be determined based on a complexity of the different anatomical structures. As a specific embodiment of the present disclosure, the image reconstruction process is described in detail below. In some embodiments, as shown in FIG. 15, the image reconstruction method includes steps S601 to S605.

In step S601, a plurality of scan regions of the scan object are acquired, and speeds of the mobile scanning device in the scan regions are determined.

In step S602, on a condition that the detector is fixed in position, the scanning bed are controlled to perform a non-uniform planar movement along a horizontal axis to obtain the raw scan data.

In step S603, on a condition that the scanning bed is fixed in position, the detector is controlled to rotate relative to the horizontal axis to obtain raw scan data.

In step S604, the raw scan data are corrected based on scan-motion-associated correction factors to obtain corrected raw scan data.

In step S605, image reconstruction is performed based on the corrected raw scan data to obtain a scan image of the scan object.

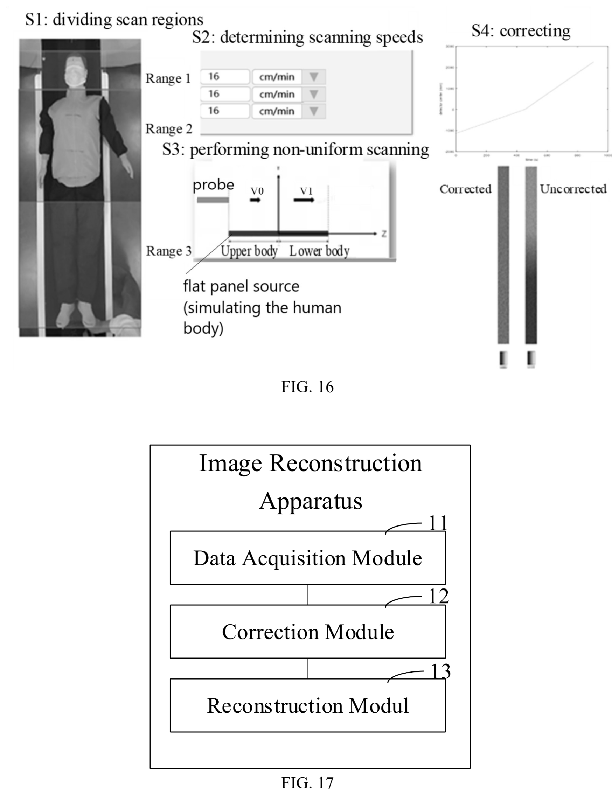

FIG. 16 is a flowchart of the image reconstruction method, which includes the following steps S1 to S4. In step S1, the scan regions of the scan object are divided. In the FIG. 16, the scan object is divided into three regions, which include range 1, range 2 and range 3. In step S2, corresponding scanning speeds for each of the scan regions are determined. In step S3, the mobile scanning device is controlled to perform non-uniform scanning based on the scanning speeds to obtain raw scan data. In step S4, the raw scan data are corrected using correction factors, and image reconstruction is performed based on the corrected raw scan data to obtain a scan image of the scan object.

It should be understood that, although the steps in the flowcharts of the above embodiments are shown in sequence as indicated by the arrows, these steps are not necessarily performed in the order indicated by the arrows. Unless otherwise specified herein, there is no strict order restriction on the execution of these steps, and these steps can be performed in other orders. Moreover, at least a portion of the steps in the flowcharts of the above embodiments may include multiple steps or multiple stages, and these steps or stages are not necessarily performed at the same time, but can be performed at different times. The execution order of these steps or stages is not necessarily to be performed in sequence, but can be performed in turn or alternately with other steps or at least a portion of steps or stages in other steps.

Based on the same inventive concept, embodiments of the present disclosure further provide an image reconstruction apparatus for implementing the aforementioned image reconstruction method. The solution provided by this device is similar to the solution described in the aforementioned method. Therefore, the specific limitations in one or more of the following embodiments of the image reconstruction apparatus can be found in the above-described limitations on the image reconstruction method and will not be further elaborated here.

In an exemplary embodiment, as shown in FIG. 17, an image reconstruction apparatus is provided. The apparatus includes a data acquisition module 11, a correction module 12, and a reconstruction module 13.

The data acquisition module 11 is configured to acquire raw scan data of a scan object by a scanning system. The scanning system includes a detector.

The correction module 12 is configured to correct the raw scan data based on at least one of scan-motion-associated correction factors to obtain corrected raw scan data. The scan-motion-associated correction factors are associated with scanning durations of a plurality of voxels of the scan object or scanning durations of a plurality of pixels of the detector.

The reconstruction module 13 is configured to perform image reconstruction based on the corrected raw scan data to obtain a scan image of the scan object.

In some embodiments, the scanning system further includes a scanning bed. The data acquisition module is further configured to control the scanning bed to perform a movement along a horizontal axis to obtain the raw scan data.

In some embodiments, the scan-motion-associated correction factors include correction factors corresponding to different voxels of the scan object. the apparatus further includes a time acquisition module and a factor determination module.

The time acquisition module is configured to acquire start scan time points and end scan time points corresponding to the scanning durations of the plurality of voxels of the scan object.

The factor determination module is configured to determine the correction factors corresponding to the voxels of the scan object based on the start scan time points and the end scan time points.

In some embodiments, the factor determination module includes a calculation unit and a factor generation unit.

The calculation unit is configured to calculate an average moving speed of the scanning bed based on the start scan time points and the end scan time points corresponding to the voxels.

The factor generation unit is configured to generate the correction factors corresponding to the voxels based on the start scan time points, the end scan time points, the average moving speed, and a nuclide half-life of a nuclide in the scan object.

In some embodiments, data acquisition module is configured to control the detector to rotate relative to a horizontal axis to obtain the raw scan data.

In some embodiments, the scan-motion-associated correction factors include correction factors corresponding to different pixels of the detector. The correction module is configured to generate a likelihood function corresponding to the raw scan data based on the scanning durations and the correction factors, and correct the raw scan data based on the likelihood function to obtain the corrected raw scan data.

In some embodiments, the correction module is configured to acquire predicted scan data corresponding to the raw scan data on a condition that the likelihood function takes a maximum value, and use the predicted scan data as the corrected raw scan data.

In some embodiments, the data acquisition module is configured to acquire a plurality of scan regions of the scan object, determine scanning durations of the scanning system for the plurality of scan regions, and perform scanning based on the scanning durations of the scanning system for the plurality of scan regions to acquire the raw scan data.

In some embodiments, the data acquisition module is configured to determine scanning moving speeds or scanning residence durations of the detector for the plurality of scan regions, based on the scanning durations of the scanning system for the plurality scan regions, and perform scanning based on the scanning moving speeds or the scanning residence durations to acquire the raw scan data.

In some embodiments, the scan regions are divided based on scanning requirements of the scan object.

In some embodiments, the scan regions are divided based on an anatomical structure of the scan object.

The above-mentioned image reconstruction apparatus may be implemented in whole or in part through software, hardware, or a combination thereof. Each module may be embedded in or independent of a processor in a computer device in the form of hardware, or may be stored in a memory in the computer device in the form of software, so that the processor can call and execute the corresponding operations of each module.

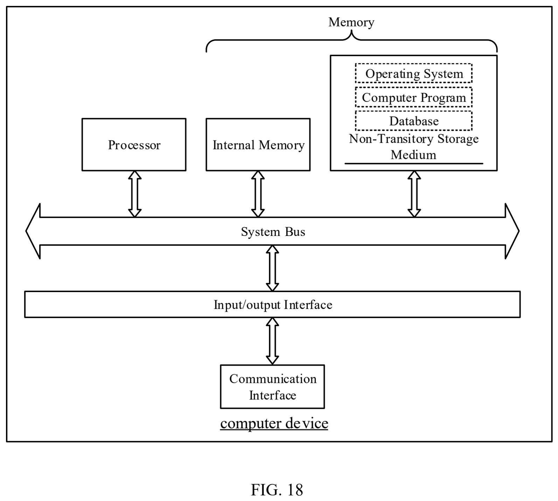

In an exemplary embodiment, a computer device is provided, which may be a server. Its internal configuration may be as shown in FIG. 18. The computer device includes a processor, memory, an input/output (I/O) interface, and a communication interface. The processor, memory, and I/O interface are connected via a system bus, and the communication interface is connected to the system bus via the I/O interface. The processor of the computer device is used to provide computing and control capabilities. The memory of the computer device includes a non-transitory storage medium and internal memory. The non-transitory storage medium stores an operating system, a computer program, and a database. The internal memory provides an environment for the operation of the operating system and computer program in the non-transitory storage medium. The database of the computer device is configured to store data during the image reconstruction process. The I/O interface of the computer device is configured to exchange information between the processor and external devices. The communication interface of the computer device is configured to communicate with external terminals via a network connection. When executed by the processor, the computer program implements an image reconstruction method.

Those skilled in the art will understand that the structure shown in FIG. 18 is merely a block diagram of a portion of a structure related to the solution of the present disclosure, and does not constitute a limitation on the computer device to which the solution of the present disclosure is applied. The specific computer device may include more or fewer components than shown in FIG. 18, or combine certain components, or have a different component arrangement.

In an exemplary embodiment, a computer device is provided. The computer device includes a memory and a processor, a computer program is stored in the memory, and when the processor executes the computer program, the steps of any one embodiment of the above-mentioned image reconstruction method are implemented.

In some embodiments, a computer-readable storage medium is provided, on which a computer program is stored. When the computer program is executed by a processor, the steps of any one embodiment of the above-mentioned image reconstruction method are implemented.

In some embodiments, a computer program product is provided. The computer program includes a computer program, which implements the steps of any one embodiment of the above-mentioned image reconstruction method when executed by a processor.

It should be noted that the user information (including but not limited to user device information, user personal information, etc.) and data (including but not limited to data used for analysis, stored data, displayed data, etc.) involved in the present disclosure are all information and data authorized by the user or fully authorized by all parties, and the collection, use and processing of relevant data must comply with relevant regulations.

Those skilled in the art will understand that all or part of the processes in the above-mentioned embodiment methods can be implemented by instructing the relevant hardware through a computer program. The computer program can be stored in a non-transitory computer-readable storage medium. When the computer program is executed, it can include the processes of the embodiments of the above-mentioned methods. Any reference to memory, database or other media used in the embodiments provided in the present disclosure may include at least one of non-transitory memory and transitory memory. Non-transitory memory may include read-only memory (ROM), magnetic tape, floppy disk, flash memory, optical memory, high-density embedded non-transitory memory, resistive random access memory (ReRAM), Magnetoresistive Random Access Memory (MRAM), Ferroelectric Random Access Memory (FRAM), Phase Change Memory (PCM), Graphene Memory, etc. Transitory memory may include Random Access Memory (RAM) or external cache memory, etc. By way of illustration and not limitation, RAM may take various forms, such as Static Random Access Memory (SRAM) or Dynamic Random Access Memory (DRAM). The database involved in the various embodiments provided herein may include at least one of a relational database and a non-relational database. Non-relational databases may include, but are not limited to, blockchain-based distributed databases. The processor involved in the various embodiments provided herein may be, but is not limited to, a general-purpose processor, a central processing unit, a graphics processing unit, a digital signal processor, a programmable logic unit, a quantum computing-based data processing logic unit, an artificial intelligence (AI) processor, etc.

The technical features of the above embodiments can be combined arbitrarily. In order to make the description concise, not all possible combinations of the technical features in the above embodiments are described. However, as long as there is no contradiction in the combination of these technical features, they should be considered to be within the scope of the present disclosure.

The above embodiments merely illustrate several implementation methods of the present disclosure. While the descriptions are relatively specific and detailed, they should not be construed as limiting the scope of the present disclosure. It should be noted that a person skilled in the art may make various modifications and improvements without departing from the spirit of the present disclosure, all of which fall within the scope of protection of the present disclosure. Therefore, the scope of protection of the present disclosure shall be determined by the appended claims.

**Claims**

1. An image reconstruction method, comprising,

acquiring raw scan data of a scan object by a scanning system, the scanning system comprising a detector;
correcting the raw scan data based on at least one of scan-motion-associated correction factors to obtain corrected raw scan data, the scan-motion-associated correction factors being associated with scanning durations of a plurality of voxels of the scan object or scanning durations of a plurality of pixels of the detector; and performing image reconstruction based on the corrected raw scan data to obtain a scan image of the scan object.

2. The image reconstruction method according to claim 1, wherein the raw scan data are acquired, on a condition that the scanning system, during a single scanning process, configures at least two different scanning durations respectively for the plurality of voxels of the scan object or the plurality pixels of the detector.

3. The image reconstruction method according to claim 2, wherein the scanning system further comprises a scanning bed, and acquiring the raw scan data of the scan object by the scanning system comprises:
controlling the scanning bed to perform a movement along a horizontal axis to obtain the raw scan data.

4. The image reconstruction method according to claim 3, wherein the movement comprises at least one of a stepwise movement and a continuous movement.

5. The image reconstruction method according to any one of claims 3 to 4, wherein the scan-motion-associated correction factors comprise correction factors corresponding to different voxels of the scan object; and
before correcting the raw scan data based on at least one of the scan-motion-associated correction factors, the method further comprises:

   acquiring start scan time points and end scan time points corresponding to the scanning durations of the plurality of voxels of the scan object; and
   determining, based on the start scan time points and the end scan time points, correction factors corresponding to the voxels of the scan object.

6. The image reconstruction method according to claim 5, wherein determining, based on the start scan time points and the end scan time points, the correction factors corresponding to the voxels of the scan object comprises:

   calculating an average moving speed of the scanning bed based on the start scan time points and the end scan time points corresponding to the voxels; and

generating the correction factors corresponding to the voxels based on the start scan time points, the end scan time points, the average moving speed, and a nuclide half-life of a nuclide in the scan object.

7. The image reconstruction method according to claim 2, wherein acquiring the raw scan data of the scan object by the scanning system comprises:
controlling the detector to rotate relative to a horizontal axis to obtain the raw scan data.

8. The image reconstruction method according to claim 7, wherein the scan-motion-associated correction factors comprise correction factors corresponding to different pixels of the detector; and
correcting the raw scan data based on the at least one of scan-motion-associated correction factors to obtain the corrected raw scan data comprises:

   generating a likelihood function corresponding to the raw scan data based on the scanning durations and the correction factors; and
   correcting the raw scan data based on the likelihood function to obtain the corrected raw scan data.

9. The image reconstruction method according to claim 8, wherein correcting the raw scan data based on the likelihood function to obtain the corrected raw scan data comprises:
acquiring, on a condition that the likelihood function takes a maximum value, predicted scan data corresponding to the raw scan data, and using the predicted scan data as the corrected raw scan data.

10. The image reconstruction method according to any one of claims 1 to 6, wherein acquiring the raw scan data of the scan object by the scanning system comprises:

   acquiring a plurality of scan regions of the scan object;
   determining scanning durations of the scanning system for the plurality of scan regions; and
   performing scanning based on the scanning durations of the scanning system for the plurality of scan regions to acquire the raw scan data.

11. The image reconstruction method according to claim 10, wherein performing scanning based on the scanning durations of the scanning system for the plurality of scan regions to acquire the raw scan data comprises:

   determining scanning moving speeds or scanning residence durations of the detector for the plurality of scan regions, based on the scanning durations of the scanning system for the plurality scan regions; and

performing scanning based on the scanning moving speeds or the scanning residence durations to acquire the raw scan data.

12. The image reconstruction method according to any one of claims 10 to 11, wherein the scan regions are divided based on scanning requirements of the scan object; or

wherein the scan regions are divided based on an anatomical structure of the scan object.

13. An image reconstruction apparatus, comprising:

a data acquisition module configured to acquire raw scan data of a scan object by a scanning system, the scanning system comprising a detector;

a correction module configured to correct the raw scan data based on at least one of scan-motion-associated correction factors to obtain corrected raw scan data, the scan-motion-associated correction factors being associated with scanning durations of a plurality of voxels of the scan object or scanning durations of a plurality of pixels of the detector; and

a reconstruction module configured to perform image reconstruction based on the corrected raw scan data to obtain a scan image of the scan object.

14. A computer device comprising a memory and a processor, wherein the memory stores a computer program, and the processor, when executing the computer program, performs the image reconstruction method according to any one of claims 1 to 12.

15. A non-transitory computer-readable storage medium having a computer program stored thereon, wherein the computer program, when executed by a processor, causes the processor to perform the image reconstruction method according to any one of claims 1 to 12.

101       102

| computer device | ↔ | scanning device |
|---|---|---|

**FIG. 1**

Acquire raw scan data of a scanned object by a scanning system. The scanning system includes a detector — S201

Correct the raw scan data based on at least one of scan-motion-associated correction factors to obtain corrected raw scan data. The scan-motion-associated correction factors are associated with scanning durations of a plurality of voxels of the scan object or scanning durations of a plurality of pixels of the detector — S202

Perform image reconstruction on the corrected raw scan data to obtain a scan image of the scan object — S203

**FIG. 2**

Y

Scanning probe

Z

Upper body    Lower body

**FIG. 3**

FIG. 4

FIG. 5

FIG. 6

Counts

FIG. 7

FIG. 8

Counts

FIG. 9

S401

Calculate an average moving speed of the scanning bed based on the start scan time points and the end scan time points corresponding to the voxels

S402

Generate the correction factors corresponding to the voxels based on the start scan time points, the end scan time points, the average moving speed, and a nuclide half-life of a nuclide in the scan object

FIG. 10

Scanning probe

FIG. 11

Generate a likelihood function corresponding to the raw scan data based on the scanning durations and the correction factors

S502

Correct the raw scan data based on the likelihood function to obtain the corrected raw scan data

S503

FIG. 12

Scanning probe

Projection i

FIG. 13

Counts

$t_{ia}$         $t_{ib}$

T

FIG. 14

| Acquire a plurality of scan regions of the scan object, and determine speeds of the mobile scanning device in the scan regions | S601 |

| On a condition that the detector is fixed in position, control the scanning bed to perform a non-uniform planar movement along a horizontal axisto obtain the raw scan data | S602 |

| On a condition that the scanning bed is fixed in position, control the detector to rotate relative to the horizontal axis to obtain raw scan data | S60 |

| Correct the raw scan data based on scan-motion-associated correction factors to obtain corrected raw scan data | S604 |

| Perform image reconstruction based on the corrected raw scan data to obtain a scan image of the scan object | S605 |

FIG. 15

FIG. 16

FIG. 17

FIG. 18

**EUROPEAN SEARCH REPORT**

Application Number

EP 25 21 2371

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | V Y PANIN ET AL: "Continuous bed motion on clinical scanner: design, data correction, and reconstruction", PHYSICS IN MEDICINE AND BIOLOGY, INSTITUTE OF PHYSICS PUBLISHING, BRISTOL GB, vol. 59, no. 20, 25 September 2014 (2014-09-25), pages 6153-6174, XP020270497, ISSN: 0031-9155, DOI: 10.1088/0031-9155/59/20/6153 [retrieved on 2014-09-25] * the whole document * ----- | 1-15 | INV. G06T12/10 |
| A | EP 3 396 630 A1 (SIEMENS MEDICAL SOLUTIONS USA INC [US]) 31 October 2018 (2018-10-31) * figures 1, 2 * * paragraph [0030] - paragraph [0032] * ----- | 1-15 | |

TECHNICAL FIELDS
SEARCHED (IPC)

G06T

The present search report has been drawn up for all claims

| Place of search | Date of completion of the search | Examiner |
|---|---|---|
| Munich | 24 February 2026 | Werling, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 2371

This annex lists the patent family members relating to the patent documents cited in  the above-mentioned European search report.
The members are as contained in the European Patent Office EDP file on
The European Patent Office is in no way liable for these particulars which are merely given for the purpose of information.

24-02-2026

| Patent document cited in search report | | Publication date | Patent family member(s) | | Publication date |
|---|---|---|---|---|---|
| EP 3396630 | A1 | 31-10-2018 | AU | 2018202569 A1 | 08-11-2018 |
| | | | CA | 3002342 A1 | 25-10-2018 |
| | | | CN | 108836375 A | 20-11-2018 |
| | | | EP | 3396630 A1 | 31-10-2018 |
| | | | HU | E059333 T2 | 28-11-2022 |
| | | | JP | 6793678 B2 | 02-12-2020 |
| | | | JP | 2018185303 A | 22-11-2018 |
| | | | KR | 20180119513 A | 02-11-2018 |
| | | | US | 2018303438 A1 | 25-10-2018 |

EPO FORM P0459

For more details about this annex : see Official Journal of the European Patent Office, No. 12/82